# EUROPEAN PATENT APPLICATION

(11) **EP 4 678 143 A1**
(43) Date of publication of application: **14.01.2026**
(21) Application number: 24187283.7
(22) Date of filing: 09.07.2024
(51) Int. Cl.: A61F 2/16

(54) **REFRACTIVE EDOF INTRAOCULAR LENS FOR CONTINUOUS VISION**

(71) Applicant: Biotech Vision Care Pvt. Ltd., 380058 Ahmedabad Gujarat (IN)
(72) Inventor: RAJU, Pedamallu Vvnsp, 380058 Ahmedabad (IN); PATEL, Hitendra, 380058 Ahmedabad (IN); PATEL, Vikram, 380058 Ahmedabad (IN); RATHOD, Vinod, 380058 Ahmedabad (IN)
(74) Representative: Cueto, Sénida

(57) **Abstract**

The present invention relates to intraocular lens. More specifically this invention is related to the intraocular lens (IOL) with continuous vision, along with extended depth of focus (EDOF) which provides continuous vision to the cataract patients from far distance to near distance objects with minimized halos and glare.

The main advantage of the present invention of novel refractive EDOF intraocular lens for continuous vision to provide extended depth of focus to get continuous vision from far distance to near distance around 40-35 cm closer distance. The haloes and glares are minimized by optimizing the spherical aberration at each zone of the anterior surface of the IOL. The fourth zone 60 degree segment design of invention is to provide balanced energy distribution at larger pupil diameter and makes the lens pupil independent for the power distribution. The lens power and its extended depth of focus are independent from the pupil size or light conditions. Further, zonal modification in the IOL can be used as a controllable switch to change the depth of focus according to the requirement, viz. from distant to near vision or distant to intermediate vision.

## Description

### FIELD OF INVENTION

The present invention relates to intraocular lens. More particularly the present invention is related to the intraocular lens (IOL) with continuous vision, extended depth of focus (EDOF), and more importantly provides near to distant vision, helps the cataract patients for achieving adequate continuous vision from far distance to near distance objects with minimized halos and glare. Further, zonal modification in the IOL can be used as a controllable switch to change the depth of focus according to the requirement, viz. from distant to near vision or distant to intermediate vision.

### BACKGROUND

Natural crystalline lens has the ability to adjust its curvature, resulting adjustable focal length according to the distance of the object to be imaged at the retina. However, after the cataract surgery, implanted IOLs perform the same job as the natural crystalline lens but with limited object distances. This is because of its fixed focal length and aperture, which cannot be adjusted according to the object distances as the natural crystalline lens does. Therefore, the person with implanted IOL, cannot see the objects which is beyond the limit of vision distance of the IOL. Thus the person using this type of IOL, has to wear prescription glasses for viewing other distance objects. For example, implanted mono-focal IOL, generally fit for viewing distant objects, hence for intermediate or near objects (for reading purposes) person has to wear prescription glasses. This is because implanted IOL has its fixed focal length and aperture as asserted above.

The present novel invention is an IOL which has an extended depth of focus (EDOF), and can be used to see the near to distant objects and allow the continuous vison without wearing prescription glasses.

The prior art document US20030199976A1 relates to a narrow profile, glare reducing, posterior chamber intraocular lens comprises an optic having an anterior surface and a posterior surface and an optical axis. The posterior surface is formed having two adjacent peri-axial, stepped imaging zones, the two imaging zones having the substantially same optical power. A transition zone between the two imaging zones preferably has a surface of continuous curvature, and shaped to reduce direct glare from light incident on the transition zone of the IOL. In variations, the transition zones are formed at the optic edge to minimize direct and indirect glare in the eye of an individual wearing the intraocular lens.

The prior art document describes the bifocal refractive lens, with an optical power difference between far distance and near distance vision 2.5 D, whereas other prior documents describe the refractive sectorial bifocal lens. These lenses do not have any revolutionary profile.

One of the prior documents describes the bifocal lens with aberration correction, which has been done through counterbalancing the aberration imposed by cornea. Whereas, few other prior documents claim the zonal refractive profile introduced in the IOL, enhances the depth of field up to 1.1 D. The another prior patent describes the use of opaque mask in IOL, having an objective to induce stenopeic effect and hence enhanced depth of field. Various techniques like bulls eye refractive principle of higher power central zones has been used earlier to extend the depth of focus. Further the patent US005864380A, document, describe the design of IOL which useful for specifically close range work, while facilitating up to distinct vision up to a specific distance. Another patent document US9201250B2, describes the design which is used in mitigation or treating the refractive error at distances encompassing far to near without significant ghosting.

Few other patent documents describe the opaque mask to induce stenopeic effect, but no inclusion of multifocal surfaces, the variable transmittance with maximum at central region and minimum at periphery of the IOL. It has been claimed in this patent that change in the transmittance allow the lens to increase the depth of field. Yet another prior art also describes the use of phase mask enhances the depth of focus up to 3.0 D. The use of opaque mask in the lOLs compromise the contrast of the image at different light conditions. All these lOLs provide limited solution as described above.

Hence all the above-mentioned prior arts fail to provide a continuous vision IOL with extended depth of focus which is essential in order to view the objects from distant to near with minimal glares and halos.

### SUMMARY OF INVENTION

The lens of invention has been proven most suitable for the cataract patients. Cataract is an eye disease in which patient vision becomes cloudy because of the natural crystalline lens losses its transparency due to aging effect. During the cataract surgery doctor remove the inner part of the crystalline lens which is responsible for the cloudy vision, and implant the IOL. The implanted IOL provides the similar vision as the natural crystalline lens of the human eye. The present invention is extended depth of focus which provides continuous vision from far distance to near distance along with intermediate distance objects with minimized glare and halos. Further, zonal modification in the IOL can be used as a controllable switch to change the depth of focus according to the requirement, viz. from distant to near vision or distant to intermediate vision.

The main objective of the present invention of refractive EDOF IOL is to provide extended depth of focus to get continuous vision from far to near distance up to closer distances.

Other main objective of the present invention of refractive EDOF- IOL is the fourth zone 60-degree segment design to provide the balanced energy distribution without compromising the depth of focus at larger pupil diameter like scotopic condition.

Another objective of the present invention is that, zonal modification in the IOL can be used as a controllable switch to change the depth of focus according to the requirement, viz. from distant to near vision or distant to intermediate vision.

Another main objective of the present invention of refractive EDOF-IOL is to minimize glares and halos by providing controlled spherical aberration at each zone.

Further this EDOF-IOL balances the nominal power or distance vision power and provides pupil independence.

Another main objective of the EDOF IOL is the optimized energy distribution in each annular zone to maintain the good contrast irrespective of the pupil size and light conditions.

Another main objective of the EDOF IOL is to provide the vision at functional intermediate distance and functional near distance at any pupil size irrespective of the light condition, which are missing the traditional multifocal IOLs and monofocal IOLs

Further this lens provides balanced light distribution and maintains the depth of focus along with the image quality and contrast due to the introduction of concentric 60 degree segmented circular zone design.

Further this EDOF-IOL anterior surface peripheral zone has been given negative curvature in order to reduce the central thickness. Reduced central thickness is useful during unfold of the lens after passing through the small incision during implantation.

### BRIEF DESCRIPTION OF DRAWINGS OF INVENTION

Figure 1 represents the anterior surface of the invention.
Figure 2 represents the posterior surface of the invention.
Figure 3 represents the main parts of the intraocular lens
Figure 4 describes the concentric annular zones in the invention.
Figure 5 describes about the 60-degree arc segments in the fourth zone represented with the part number 7 in figure 4 of the invention.
Figure 6 describes about the posterior surface square edge of EDOF IOL design.
Figure 7 & Figure 8 explains about the power distribution/ optical output result (autofocus scan) of the invention with optimized circular zones.

### DETAILED DESCRIPTION OF INVENTION

The following paragraphs describes about the design of EDOF IOL and its outcomes and advantages of the design. The subject matter is explained with the drawings at required places, where in like reference numerals are used.

The present invention of intraocular lens is called extended depth of focus Intraocular lens (EDOF IOL) to provide the continuous vision. This lens offers clear vision from far distance to near distance up to closer distances. This new additional feature is providing a near vision along with intermediate vision. Further, zonal modification in the IOL can be used as a controllable switch to change the depth of focus according to the requirement, viz. from distant to near vision or distant to intermediate vision.

The refractive EDOF property is achieved based on the principle of refraction. Refraction is the bending of light ray when passing through one medium to another medium. The bending of light rays is proportional to the curvature of the medium, in which light rays are entering and exit from the medium. Based on this principle the present invention is developed.

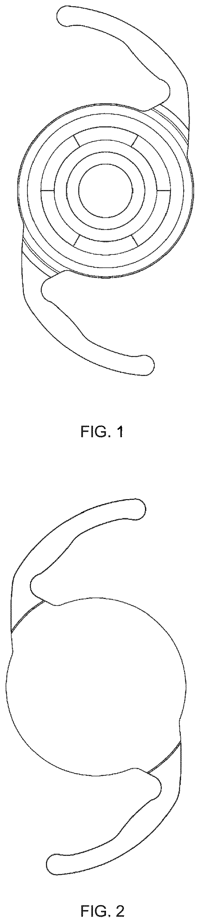

The present invention refractive EDOF IOL is related to intraocular lenses, which are used in the cataract surgery. This refractive EDOF lens is developed with hydrophobic or hydrophilic acrylic material having the refractive index of 1.40-1.6. The EDOF intraocular lens design is having single piece structure as described in the figure 1. The front surface of the intraocular lens, called, anterior surface of the invention. This surface is the main objective of this invention, having the controllable refractive circular zones and 60 degree refractive segments in one of the concentric refractive circular zone to provide the continuous vision. The posterior surface of the invention is represented by figure 2, have the single radius of curvature for entire posterior surface.

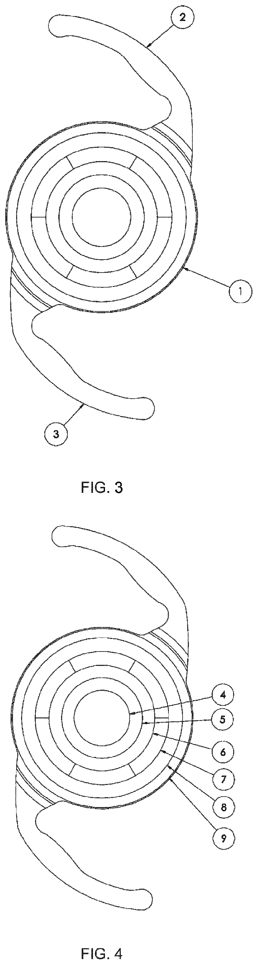

The part number 1 in the figure 3 represents the optic portion of the intraocular lens, which is responsible for the light ray refraction and continuous vision inside the eye. The part number 2 & 3 represents the modified C type haptics of the intraocular lens. The modified C type haptics provides maximum contact angle with the capsular bag, which provides the better rotational stability in the eye.

Figure 4 shows the center optic portion of the intraocular lens having the innovative design to provide extended depth of focus to see the objects from far distance to near distance with continuous vision. The center optic portion having five concentric annular refractive zones represented with the part numbers 4 to 9 as shown in figure 4 with in the 4.7 mm clear optic zone on the anterior surface of the optic portion. The peripheral zone as shown in part number 9 has been given negative curvature to the IOL anterior surface to control the thickness of the lens to fold and unfold easily during the implantation of the IOL by using micro incision process.

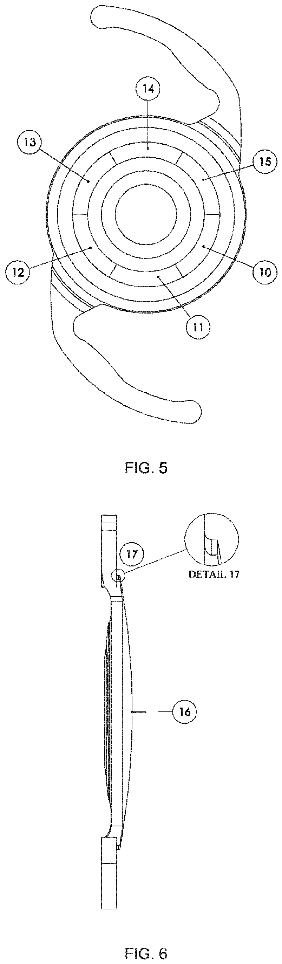

The part numbers 11 to 15 shown in figure 5 represents the 60 degree refractive segments in the fourth zone from the center of the invention to balance the energy distribution at larger pupil diameters as shown in the figure 4 part number 7. By, zonal modification in the IOL can be used as a controllable switch to change the depth of focus according to the requirement, viz. from distant to near vision or distant to intermediate vision.

Spherical aberration of the lens plays very important role in the optics. When light rays passing through the lens system, paraxial light rays and marginal light rays focus at different focal points on the optic axis, instead of focusing at a single point. In case of negative spherical aberration, marginal light rays refract less and focus far away from the lens surface and in the positive spherical aberration, marginal light rays refract more and focus near by the lens surface. By using this principle, the energy is distributed between the required powers uniformly and provides the extended depth of focus continuously with reduced glare and halos.

The part number 16 in figure 6 represents the posterior surface side view. The part number 17 represents the 360 degree posterior surface square edge of the intraocular lens. The design of the square edge will provide proper shrinkage of the capsular bag with the posterior surface of the intraocular lens and prevent the early stage PCO (Posterior Capsular Opacification).

In the design and development of invention, Hydrophobic or hydrophilic materials are used with variable range of refractive index 1.4-1.6. During the development of the invention, the size of the optic portion has been taken 6 mm with 13.0 mm overall diameter. The optic size and overall diameter of the proposed IOL can be varied as per requirement and it won't affect the optical performance of the depth of focus of the IOL. By using above mentioned methodology, around 3.5 D depth of focus has been achieved by optimizing the zone radius and power distribution on the concentric circular zones. The optimization of the zone diameters and power distribution on the concentric circular zones allow us to achieve desired depth of focus for intermediate distance to near distance visual activities along with far distance.

### Optical Material: Hydrophobic or Hydrophilic material

Refractive Index: 1.40-1.6
Optic Surface: Aspheric
Optical Size of the IOL: 6 mm
Number of concentric refractive circular zones in the clear optic area: 5 (in the clear optic zone).

### RESULTS OF THE PRESENT INVENTION

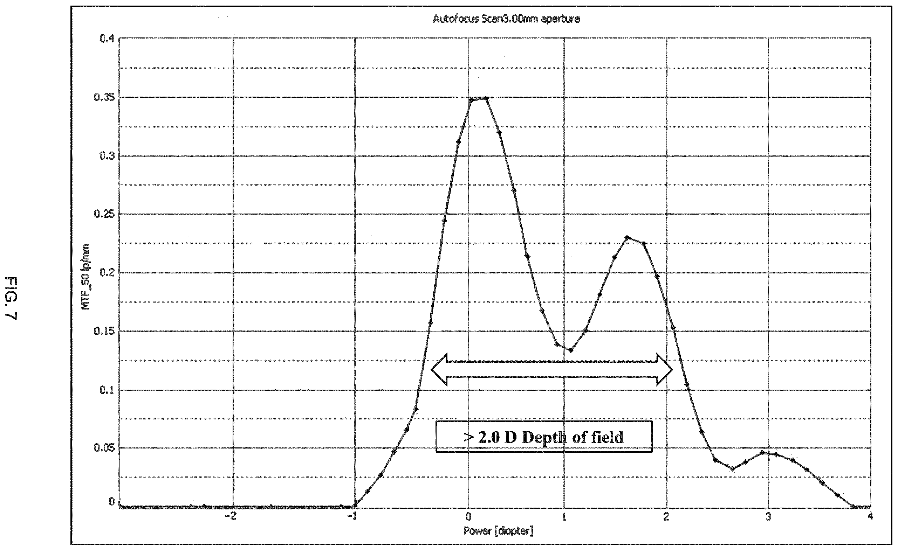

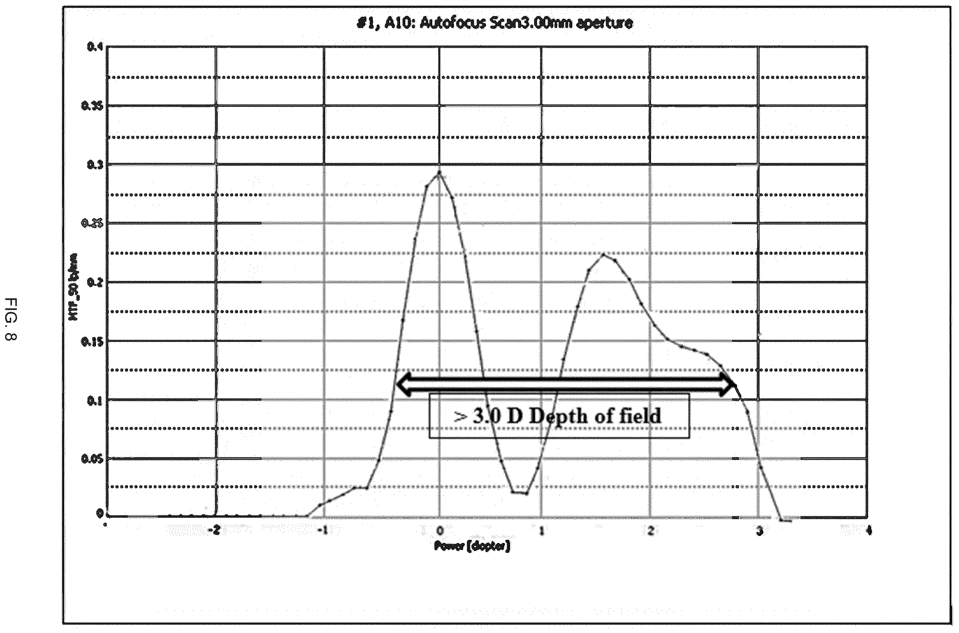

The invention of the Refractive EDOF IOL gives satisfactory results on the optical bench performance. Continuous and extended depth of focus observed in the output result from 1.0 D to 3.5 D. Figure 7 represents the more than 2.0 D depth of field of the of the intraocular lens by using Modulation Transfer Function (MTF) curve and figure 8 represents the more than 3.0 D depth of field of the intraocular lens by using MTF curve. The Modulation Transfer Function (MTF) curve in the fig 7 & 8 shows the energy distribution of the IOL at 3 mm aperture size. The achieved depth of field is useful to see the objects from far distance to intermediate distance as shown in the figure 7 and far distance to near distance as shown in the figure 8 for daily activities.

The present invention relates to intraocular lens (IOL) with extended depth of focus, which helps in providing adequate vision for far objects to near objects with minimized glares and halos. The main advantage of the present invention refractive EDOF intraocular lens is to provide extended depth of focus to get continuous vision from far distance to near distance. The optimized power distribution on each concentric circular zone and controlled zone width provides the continuous range of vision for daily activities. Controlled spherical aberration and 60 degree refractive segments in the fourth zone makes the nominal power independent from the pupil size (eye pupil) and minimize haloes and glares in the visual acuity of the patient after implanting the lens in the eye.

## Claims

1. A Novel refractive EDOF intraocular lens for continuous vision, comprising of; a posterior segment having a single radius curvature for entire posterior surface (16) wherein the posterior segment having a 360-degree square edge (17) to prevent the early PCO (Posterior Capsular Opacification); an anterior segment comprising of refractive circular zones (1) wherein a peripheral zone (9) having a negative curvature to control thickness of lens to fold and unfold easily; an option portion for ray diffraction and continuous vision inside an eye; a modified C type haptics (2,3) providing maximum contact angle with capsular bags for better rotational stability of the eye; a center optic portion comprising of five concentric annual refractive zones (4,5,6,7,8), **characterized in that**; a fourth zone (7) from center of the intraocular lens comprises of 60-degree refractive segments (10, 11, 12, 13, 14, 15) to balance the energy distribution at larger pupil diameters; a zonal modification in the intraocular lens is a controllable switch to change depth of focus.

2. The Novel refractive EDOF intraocular lens for continuous vision according to claim 1, wherein the size of optic portion is 6 mm and the overall diameter of intraocular lens is 13.0 mm.

3. The Novel refractive EDOF intraocular lens for continuous vision according to claim 1, wherein the intraocular lens is selected from hydrophobic or hydrophilic acrylic material having refractive index 1.40-1.6.

4. The Novel refractive EDOF intraocular lens for continuous vision according to claim 1, wherein the intraocular lens minimizes glare and halos by providing controlled spherical aberration at each refractive zone (4,5,6,7,8).

5. The Novel refractive EDOF intraocular lens for continuous vision according to claim 1, wherein a negative spherical aberration and a positive spherical aberration distributes energy between required powers uniformly and provides extended depth of focus continuously.

6. The Novel refractive EDOF intraocular lens for continuous vision according to claim 1, wherein the intraocular lens balances the nominal power or distant vision power and provides pupil independence along with continuous vision.

7. The Novel refractive EDOF intraocular lens for continuous vision according to claim 1, wherein the annual refractive zones (4,5,6,7,8) comprises of optimized energy distribution to maintain good contrast irrespective of pupil size and light condition along with the continuous vision.
